# EUROPEAN PATENT APPLICATION

(11) **EP 4 600 927 A1**
(43) Date of publication of application: **13.08.2025**
(21) Application number: 24806093.1
(22) Date of filing: 29.01.2024
(51) Int. Cl.: G08B 21/24, A61B 5/11, A61B 5/00

(54) **FATIGUE REMINDING METHOD AND APPARATUS, AND ELECTRONIC DEVICE**

(30) Priority: 15.05.2023 CN 202310547758
(71) Applicant: Huawei Technologies Co., Ltd., Shenzhen, Guangdong 518129 (CN)
(72) Inventor: LI, Xin, Shenzhen, Guangdong 518129 (CN); CHEN, Xiaohan, Shenzhen, Guangdong 518129 (CN); LIU, Hang, Shenzhen, Guangdong 518129 (CN); WANG, Chuhuai, Guangzhou, Guangdong 510062 (CN); YANG, Jiajia, Guangzhou, Guangdong 510062 (CN); HAO, Zengming, Guangzhou, Guangdong 510062 (CN)
(74) Representative: Goddar, Heinz J.
(86) International application number: PCT/CN2024/074363
(87) International publication number: WO 2024/234730

(57) **Abstract**

A fatigue alerting method and apparatus, and an electronic device are provided. The fatigue alerting method includes: An electronic device detects a current head posture angle of a user, and obtains duration in which the user is at the current head posture angle (S201); calculates a current cervical spine fatigue index of the user based on a calculation parameter (S202); and gives a fatigue alert to the user based on the cervical spine fatigue index (S203). According to the fatigue alerting method, the head posture angle of the user can be detected, the cervical spine fatigue index of the user can be determined with reference to the duration in which the user is at the current head posture angle, and then the fatigue alert can be given to the user based on the cervical spine fatigue index of the user, so that fatigue alerting is triggered more scientifically.

## Description

This application claims priority to Chinese Patent Application No. 202310547758.6, filed with the China National Intellectual Property Administration on May 15, 2023 and entitled "FATIGUE ALERTING METHOD AND APPARATUS, AND ELECTRONIC DEVICE", which is incorporated herein by reference in its entirety.

### TECHNICAL FIELD

Embodiments of this application relate to the field of smart terminal technologies, and in particular, to a fatigue alerting method and apparatus, and an electronic device.

### BACKGROUND

With popularization of electronic devices in the communication era, the incidence of neck pain increases rapidly and the younger are prone to the neck pain. Long-term head bowing and desk work lead to neck muscle strain, aggravating the neck pain and cervical spondylosis. Real-time head posture detection and timely alerting become key means to prevent cervical spine pain and chronic strain. The detection means are mature, but a scientific and appropriate alerting solution is still in an exploration stage.

### SUMMARY

Embodiments of this application provide a fatigue alerting method and apparatus, and an electronic device, and embodiments of this application further provide a computer-readable storage medium, to detect a head posture angle of a user, determine a cervical spine fatigue index of the user with reference to duration in which the user is at the current head posture angle, and then give a fatigue alert to the user based on the cervical spine fatigue index of the user, so that fatigue alerting is triggered more scientifically.

According to a first aspect, an embodiment of this application provides a fatigue alerting method, including: detecting, by an electronic device, a current head posture angle of a user, and obtaining duration in which the user is at the current head posture angle; calculating a current cervical spine fatigue index of the user based on a calculation parameter, where the calculation parameter includes the current head posture angle of the user and the duration; and giving a fatigue alert to the user based on the cervical spine fatigue index.

In the foregoing fatigue alerting method, the electronic device detects the current head posture angle of the user, obtains the duration in which the user is at the current head posture angle, then calculates the current cervical spine fatigue index of the user based on the calculation parameter, and gives a fatigue alert to the user based on the cervical spine fatigue index. In this way, the head posture angle of the user can be detected, the cervical spine fatigue index of the user is determined with reference to the duration in which the user is at the current head posture angle, and the fatigue alert can be given to the user based on the cervical spine fatigue index of the user, so that fatigue alerting is triggered more scientifically.

In a possible implementation, the electronic device includes a head-wearable device worn by the user, and the head-wearable device includes an inertial sensor. The detecting a current head posture angle of a user includes: obtaining inertial sensor data and determining the current head posture angle of the user based on the inertial sensor data.

In a possible implementation, the detecting a current head posture angle of a user includes: obtaining data of a user head image photographed by a camera, where the camera is disposed in the electronic device, or the camera is communicatively connected to the electronic device; and determining the current head posture angle of the user based on the data of the user head image.

In a possible implementation, the calculation parameter further includes basic information of the user, and the basic information of the user includes one or a combination of the following: an age, a gender, a height, and a weight of the user.

In a possible implementation, the detecting a current head posture angle of a user includes: periodically detecting the current head posture angle of the user; and the obtaining duration in which the user is at the current head posture angle includes: calculating a difference between a head posture angle detected in a current period and a head posture angle detected in a previous period; and if the difference is less than or equal to a predetermined threshold, increasing the duration in which the user is at the current head posture angle by duration of the current period.

In a possible implementation, the giving a fatigue alert to the user based on the cervical spine fatigue index includes: when the cervical spine fatigue index is greater than or equal to a first fatigue threshold and less than a second fatigue threshold, giving an alert of mild cervical spine fatigue to the user, and recording a first moment at which the alert of mild cervical spine fatigue is given; when the cervical spine fatigue index is greater than or equal to the second fatigue threshold and less than a third fatigue threshold, and a time interval between a current moment and the first moment is greater than or equal to a predetermined time interval, giving an alert of moderate cervical spine fatigue to the user, and recording a second moment at which the alert of moderate cervical spine fatigue is given; and when the cervical spine fatigue index is greater than or equal to the third fatigue threshold and less than a fourth fatigue threshold, and a time interval between a current moment and the second moment is greater than or equal to a predetermined time interval, giving an alert of severe cervical spine fatigue to the user, and recording a third moment at which the alert of severe cervical spine fatigue is given.

In a possible implementation, after the giving a fatigue alert to the user based on the cervical spine fatigue index, the method further includes: in response to a trigger operation of the user, playing voice or video guidance of a cervical spine relaxation action.

According to a second aspect, an embodiment of this application provides an electronic device. The electronic device has a function of implementing behavior of the electronic device in the first aspect and the possible implementations of the first aspect. The function may be implemented by using hardware, or may be implemented by hardware executing corresponding software. The hardware or the software includes one or more modules or units corresponding to the foregoing functions. For example, a detection module, a calculation module, and an alerting module.

According to a third aspect, an embodiment of this application provides an electronic device, including one or more processors, a memory, a plurality of applications, and one or more computer programs. The one or more computer programs are stored in the memory, the one or more computer programs include instructions, and when the instructions are executed by the electronic device, the electronic device is enabled to perform the method provided in the first aspect.

It should be understood that technical solutions of the second aspect and the third aspect of embodiments of this application are consistent with technical solutions of the first aspect of embodiments of this application, and beneficial effect achieved by the aspects and corresponding feasible implementations are similar. Details are not described again.

According to a fourth aspect, an embodiment of this application provides a computer-readable storage medium. The computer-readable storage medium stores a computer program. When the computer program is run on a computer, the computer is enabled to perform the method provided in the first aspect.

According to a fifth aspect, an embodiment of this application provides a computer program. When the computer program is executed by a computer, the computer program is used to perform the method in the first aspect.

In a possible design, all or a part of the program in the fifth aspect may be stored in a storage medium that is packaged together with a processor, or may be stored in a memory that is not packaged together with a processor.

### BRIEF DESCRIPTION OF DRAWINGS

FIG. 1 is a diagram of a structure of an electronic device according to an embodiment of this application;
FIG. 2 is a flowchart of a fatigue alerting method according to an embodiment of this application;
FIG. 3 is a flowchart of a fatigue alerting method according to another embodiment of this application;
FIG. 4 is a flowchart of a fatigue alerting method according to another embodiment of this application;
FIG. 5 is a flowchart of a fatigue alerting method according to another embodiment of this application;
FIG. 6 is a flowchart of a fatigue alerting method according to another embodiment of this application;
FIG. 7 is a diagram of a structure of an electronic device according to another embodiment of this application; and
FIG. 8 is a diagram of a structure of an electronic device according to another embodiment of this application.

### DESCRIPTION OF EMBODIMENTS

Terms used in embodiments of this application are only used to explain specific embodiments of this application, but are not intended to limit this application.

In an existing head bowing alerting or cervical spine alerting solution, a real-time posture angle of a head is generally obtained by using a head-mounted device or an external camera, and threshold determining is performed on the posture angle. If the angle exceeds a threshold, it is determined that the head is bowed, and if the angle does not exceed the threshold, it is determined that the head is not bowed. When accumulated duration of head bowing exceeds set time, an alert is given. After the alert is given, resetting is performed and duration of the head bowing is accumulated again.

However, the foregoing solution has the following disadvantages:
(1) Impact of different head bowing angles on cervical spine fatigue is not considered. Only a single threshold is used to determine whether the head is bowed or not.
(2) The accumulated duration of the head bowing is single. A system defaults or a user selects the duration. After the duration is selected, the accumulated duration is fixed and accumulated duration under different head bowing angles is the same.
(3) Current cervical spine health statuses of different users are not considered. An alerting mechanism for all users is the same.
(4) After the alert is given, the accumulated duration for the head bowing is reset, and whether the user relaxes subsequently is not determined.

In conclusion, in the head lowering alerting solution or the cervical spine alerting solution provided in the conventional technology, a scientific alert cannot be given to the user when the user actually encounters cervical spine fatigue.

Based on the foregoing problem, an embodiment of this application provides a fatigue alerting method. A solution of determining a cervical spine fatigue degree of a user based on head bowing time, a head bowing angle, and a personal basic indicator of the user, and give an alert in time is provided based on scientific experiments. In the fatigue alerting method provided in embodiments of this application, different accumulation degrees of cervical spine fatigue at different angles are considered for triggering fatigue alerting, a fatigue alerting time point may be adjusted, and for people in different cervical spine health statuses, an alert triggering time point may be adjusted. In addition, after the fatigue alert is given, a head status of a user may be continuously monitored, and a multi-level alert is given.

The fatigue alerting method provided in embodiments of this application may be applied to an electronic device. The electronic device may be a device like a smartphone, a smart television, a tablet computer, a wearable device, a vehicle-mounted device, an augmented reality (augmented reality, AR)/virtual reality (virtual reality, VR) device, a notebook computer, an ultra-mobile personal computer (ultra-mobile personal computer, UMPC), a netbook, or a personal digital assistant (personal digital assistant, PDA). The wearable device may include a head-wearable device provided with an inertial measurement unit (inertial measurement unit, IMU), for example, a smart helmet, smart glasses, a smart headset, or the like. A specific type of the electronic device is not limited in embodiments of this application.

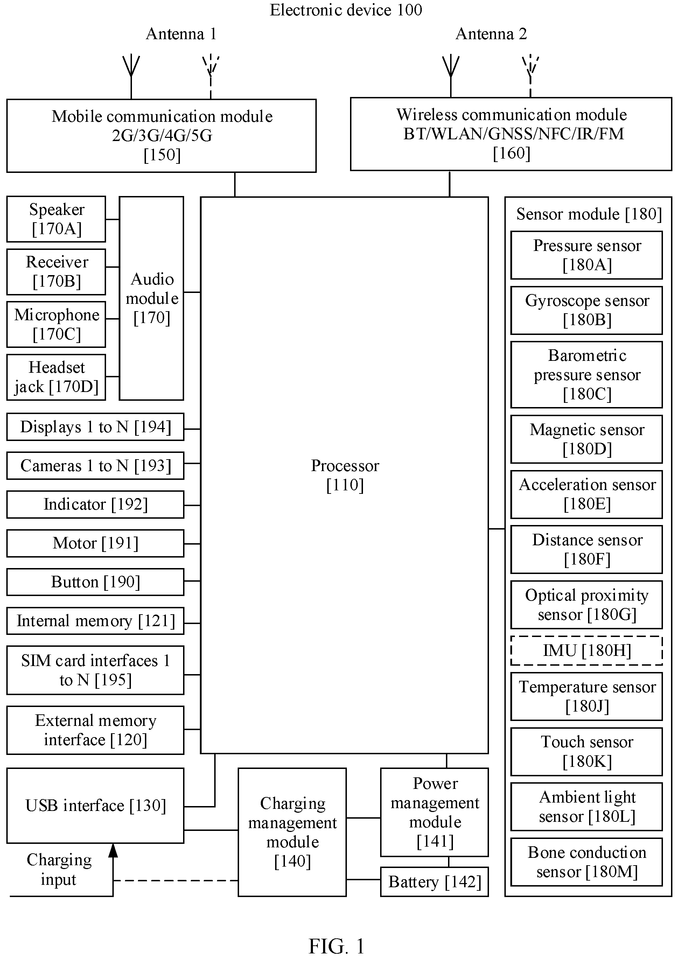

For example, FIG. 1 is a diagram of a structure of an electronic device according to an embodiment of this application. As shown in FIG. 1, the electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a temperature sensor 180J, a touch sensor 180K, an ambient light sensor 180L, a bone conduction sensor 180M, and the like. In addition, when the electronic device 100 is a head-wearable device, the sensor module 180 may further include an IMU 180H.

It may be understood that the structure shown in this embodiment of this application does not constitute a specific limitation on the electronic device 100. In some other embodiments of this application, the electronic device 100 may include more or fewer components than those shown in the figure, or combine some components, or split some components, or have different component arrangements. The components shown in the figure may be implemented by hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on instruction operation code and a time sequence signal, to control instruction fetching and instruction execution.

A memory may be further disposed in the processor 110, and is configured to store instructions and data. In some embodiments, the memory in the processor 110 is a cache memory. The memory may store instructions or data that has been used or cyclically used by the processor 110. If the processor 110 needs to use the instructions or the data again, the processor 110 may directly invoke the instructions or the data from the memory. This avoids repeated access and reduces waiting time of the processor 110, thereby improving system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a two-way synchronization serial bus, and includes one serial data line (serial data line, SDL) and one serial clock line (serial clock line, SCL). In some embodiments, the processor 110 may include a plurality of groups of I2C buses. The processor 110 may be separately coupled to the touch sensor 180K, a charger, a flash, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The I2S interface may be configured to perform audio communication. In some embodiments, the processor 110 may include a plurality of groups of I2S buses. The processor 110 may be coupled to the audio module 170 through the I2S bus, to implement communication between the processor 110 and the audio module 170. In some embodiments, the audio module 170 may transmit an audio signal to the wireless communication module 160 through the I2S interface, to implement a function of answering a call through a Bluetooth headset.

The PCM interface may also be used to perform audio communication, and sample, quantize, and code an analog signal. In some embodiments, the audio module 170 may be coupled to the wireless communication module 160 through a PCM bus interface. In some embodiments, the audio module 170 may also transmit an audio signal to the wireless communication module 160 through the PCM interface, to implement a function of answering a call through a Bluetooth headset. Both the I2S interface and the PCM interface may be used for audio communication.

The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 110 to the wireless communication module 160. For example, the processor 110 communicates with a Bluetooth module in the wireless communication module 160 through the UART interface, to implement a Bluetooth function. In some embodiments, the audio module 170 may transmit an audio signal to the wireless communication module 160 through the UART interface, to implement a function of playing music through a Bluetooth headset.

The MIPI interface may be configured to connect the processor 110 to a peripheral component like the display 194 or the camera 193. The MIPI interface includes a camera serial interface (camera serial interface, CSI), a display serial interface (display serial interface, DSI), and the like. In some embodiments, the processor 110 communicates with the camera 193 through the CSI, to implement a photographing function of the electronic device 100. The processor 110 communicates with the display 194 through the DSI interface, to implement a display function of the electronic device 100.

The GPIO interface may be configured by software. The GPIO interface may be configured as a control signal or a data signal. In some embodiments, the GPIO interface may be configured to connect the processor 110 to the camera 193, the display 194, the wireless communication module 160, the audio module 170, the sensor module 180, or the like. The GPIO interface may alternatively be configured as an I2C interface, an I2S interface, a UART interface, an MIPI interface, or the like.

The USB interface 130 is an interface that conforms to a USB standard specification, and may be specifically a mini USB interface, a micro USB interface, a USB type-C interface, or the like. The USB interface 130 may be configured to connect to a charger to charge the electronic device 100, or may be configured to transmit data between the electronic device 100 and a peripheral device, or may be configured to connect to a headset for playing audio through the headset. The interface may be further configured to connect to another electronic device like an AR device.

It may be understood that an interface connection relationship between the modules illustrated in embodiments of this application is merely an example for description, and does not constitute a limitation on the structure of the electronic device 100. In some other embodiments of this application, the electronic device 100 may alternatively use an interface connection manner different from that in the foregoing embodiment, or use a combination of a plurality of interface connection manners.

The charging management module 140 is configured to receive a charging input from the charger. The charger may be a wireless charger or a wired charger. In some embodiments of wired charging, the charging management module 140 may receive a charging input of a wired charger through the USB interface 130. In some embodiments of wireless charging, the charging management module 140 may receive a wireless charging input through a wireless charging coil of the electronic device 100. When charging the battery 142, the charging management module 140 may further supply power to the electronic device 100 by using the power management module 141.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like. The power management module 141 may be further configured to monitor parameters such as a battery capacity, a battery cycle count, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communication function of the electronic device 100 may be implemented through the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna in the electronic device 100 may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution that is applied to the electronic device 100 and that includes 2G/3G/4G/5G or the like. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules in the mobile communication module 150 may be disposed in the processor 110. In some embodiments, at least some functional modules of the mobile communication module 150 may be disposed in a same device as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent component. In some other embodiments, the modem processor may be independent of the processor 110, and is disposed in a same device as the mobile communication module 150 or another functional module.

The wireless communication module 160 may provide a wireless communication solution that is applied to the electronic device 100 and that includes a wireless local area network (wireless local area network, WLAN) (for example, a wireless fidelity (wireless fidelity, Wi-Fi) network), Bluetooth (bluetooth, BT), a global navigation satellite system (global navigation satellite system, GNSS), frequency modulation (frequency modulation, FM), a near field communication (near field communication, NFC) technology, an infrared (infrared, IR) technology, or the like. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

In some embodiments, the antenna 1 and the mobile communication module 150 in the electronic device 100 are coupled, and the antenna 2 and the wireless communication module 160 in the electronic device 100 are coupled, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-CDMA), long term evolution (long term evolution, LTE), BT, a GNSS, a WLAN, NFC, FM, an IR technology, and/or the like. The GNSS may include a global positioning system (global positioning system, GPS), a global navigation satellite system (global navigation satellite system, GLONASS), a BeiDou navigation satellite system (beidou navigation satellite system, BDS), a quasi-zenith satellite system (quasi-zenith satellite system, QZSS), and/or a satellite based augmentation system (satellite based augmentation systems, SBAS).

The electronic device 100 may implement a display function through the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. The display panel may use a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light-emitting diode (quantum dot light-emitting diode, QLED), and the like. In some embodiments, the electronic device 100 may include one or N displays 194, where N is a positive integer greater than 1.

The electronic device 100 may implement a photographing function through the camera 193, the ISP, the video codec, the GPU, the display 194, the application processor, and the like.

The ISP is configured to process data fed back by the camera 193. For example, during photographing, a shutter is pressed, and light is transmitted to a photosensitive element of the camera through a lens. An optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated through the lens, and is projected onto the photosensitive element. The photosensitive element may be a charge coupled device (charge coupled device, CCD) or a complementary metal-oxide-semiconductor (complementary metal-oxide-semiconductor, CMOS) phototransistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV. In some embodiments, the electronic device 100 may include one or N cameras 193, where N is a positive integer greater than 1.

The digital signal processor is configured to process a digital signal, and may process another digital signal in addition to the digital image signal. For example, when the electronic device 100 selects a frequency, the digital signal processor is configured to perform Fourier transformation on frequency energy.

The video codec is configured to compress or decompress a digital video. The electronic device 100 may support one or more video codecs. In this way, the electronic device 100 may play back or record videos in a plurality of coding formats, for example, moving picture experts group (moving picture experts group, MPEG)-1, MPEG-2, MPEG-3, and MPEG-4.

The NPU is a neural-network (neural-network, NN) computing processor, quickly processes input information by referring to a structure of a biological neural network, for example, by referring to a mode of transfer between human brain neurons, and may further continuously perform self-learning. Applications such as intelligent cognition of the electronic device 100 may be implemented through the NPU, for example, image recognition, facial recognition, speech recognition, and text understanding.

The external memory interface 120 may be used to connect to an external storage card, for example, a micro SD card, to extend a storage capability of the electronic device 100. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a sound playing function or an image playing function), and the like. The data storage area may store data (such as audio data and an address book) created during use of the electronic device 100, and the like. In addition, the internal memory 121 may include a high-speed random access memory, or may include a nonvolatile memory, for example, at least one magnetic disk storage device, a flash memory, or a universal flash storage (universal flash storage, UFS). The processor 110 runs instructions stored in the internal memory 121 and/or instructions stored in the memory disposed in the processor, to perform various function applications and data processing of the electronic device 100.

The electronic device 100 may implement an audio function by using the audio module 170, the speaker 170A, the receiver 170B, the microphone 170C, the headset jack 170D, the application processor, for example, music playing and recording.

The audio module 170 is configured to convert digital audio information into an analog audio signal for output, and is also configured to convert analog audio input into a digital audio signal. The audio module 170 may be further configured to encode and decode an audio signal. In some embodiments, the audio module 170 may be disposed in the processor 110, or some functional modules in the audio module 170 are disposed in the processor 110.

The speaker 170A, also referred to as a "loudspeaker", is configured to convert an audio electrical signal into a sound signal. The electronic device 100 may be used to listen to music or answer a call in a hands-free mode over the speaker 170A.

The receiver 170B, also referred to as an "earpiece", is configured to convert an audio electrical signal into a sound signal. When a call is answered or voice information is received through the electronic device 100, the receiver 170B may be put close to a human ear to listen to a voice.

The microphone 170C, also referred to as a "mike" or a "mic", is configured to convert a sound signal into an electrical signal. When making a call or sending a voice message, a user may make a sound near the microphone 170C through the mouth of the user, to input a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be the USB interface 130, or may be a 3.5 mm open mobile terminal platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and can convert the pressure signal into an electrical signal. In some embodiments, the pressure sensor 180A may be disposed on the display 194. There are a plurality of types of pressure sensors 180A, such as a resistive pressure sensor, an inductive pressure sensor, and a capacitive pressure sensor. The capacitive pressure sensor may include at least two parallel plates made of conductive materials. When a force is applied to the pressure sensor 180A, capacitance between electrodes changes. The electronic device 100 determines pressure intensity based on the change in the capacitance. When a touch operation is performed on the display 194, the electronic device 100 detects intensity of the touch operation through the pressure sensor 180A. The electronic device 100 may also calculate a touch location based on a detection signal of the pressure sensor 180A. In some embodiments, touch operations that are performed in a same touch location but have different touch operation intensity may correspond to different operation instructions. For example, when a touch operation whose touch operation intensity is less than a first pressure threshold is performed on a messages application icon, an instruction for viewing an SMS message is performed. When a touch operation whose touch operation intensity is greater than or equal to the first pressure threshold is performed on the messages application icon, an instruction for creating a new SMS message is performed.

The gyroscope sensor 180B may be configured to determine a moving posture of the electronic device 100. In some embodiments, an angular velocity of the electronic device 100 around three axes (namely, axes x, y, and z) may be determined through the gyroscope sensor 180B. The gyroscope sensor 180B may be configured to implement image stabilization during photographing. For example, when the shutter is pressed, the gyroscope sensor 180B detects an angle at which the electronic device 100 jitters, calculates, based on the angle, a distance for which a lens module needs to compensate, and allows the lens to cancel the jitter of the electronic device 100 through reverse motion, to implement image stabilization. The gyroscope sensor 180B may also be used in a navigation scenario and a somatic game scenario.

The barometric pressure sensor 180C is configured to measure barometric pressure. In some embodiments, the electronic device 100 calculates an altitude through the barometric pressure measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a clamshell phone, the electronic device 100 may detect opening and closing of a flip cover based on the magnetic sensor 180D. Further, a feature like automatic unlocking of the flip cover is set based on a detected opening or closing state of the leather case or a detected opening or closing state of the flip cover.

The acceleration sensor 180E may detect accelerations in various directions (usually on three axes) of the electronic device 100. When the electronic device 100 is still, a magnitude and a direction of gravity may be detected. The acceleration sensor 180E may be further configured to identify a posture of the electronic device, and is used in an application such as switching between a landscape mode and a portrait mode or a pedometer.

The distance sensor 180F is configured to measure a distance. The electronic device 100 may measure the distance in an infrared manner or a laser manner. In some embodiments, in a photographing scenario, the electronic device 100 may measure a distance through the distance sensor 180F to implement quick focusing.

The optical proximity sensor 180G may include, for example, a light-emitting diode (LED) and an optical detector, for example, a photodiode. The light-emitting diode may be an infrared light-emitting diode. The electronic device 100 emits infrared light by using the light-emitting diode. The electronic device 100 detects infrared reflected light from a nearby object through the photodiode. When sufficient reflected light is detected, it may be determined that there is an object near the electronic device 100. When insufficient reflected light is detected, the electronic device 100 may determine that there is no object near the electronic device 100. The electronic device 100 may detect, by using the optical proximity sensor 180G, that the user holds the electronic device 100 close to an ear for a call, to automatically turn off a screen for power saving. The optical proximity sensor 180G may also be used in a smart cover mode or a pocket mode to automatically perform screen unlocking or locking.

The ambient light sensor 180L is configured to sense ambient light brightness. The electronic device 100 may adaptively adjust brightness of the display 194 based on the sensed ambient light brightness. The ambient light sensor 180L may also be configured to automatically adjust white balance during photographing. The ambient light sensor 180L may also cooperate with the optical proximity sensor 180G to detect whether the electronic device 100 is in a pocket, to avoid accidental touch.

The inertia measurement unit (inertial measurement unit, IMU) 180H, also referred to as an inertia sensor, is a sensor configured to detect and measure an acceleration and rotation motion. In this embodiment of this application, after obtaining data detected by the IMU 180H (referred to as inertia sensor data), the processor 110 may detect a current head posture angle of a user based on the inertia sensor data.

The fingerprint sensor is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 lowers performance of a processor nearby the temperature sensor 180J, to reduce power consumption for thermal protection. In some other embodiments, when the temperature is less than another threshold, the electronic device 100 heats the battery 142 to prevent the electronic device 100 from being shut down abnormally due to a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a "touch component". The touch sensor 180K may be disposed on the display 194, and the touch sensor 180K and the display 194 form a touchscreen, which is also referred to as a "touch screen". The touch sensor 180K is configured to detect a touch operation performed on or near the touch sensor. The touch sensor may transfer the detected touch operation to the application processor to determine a type of a touch event. A visual output related to the touch operation may be provided through the display 194. In some other embodiments, the touch sensor 180K may alternatively be disposed on a surface of the electronic device 100 at a location different from that of the display 194.

The bone conduction sensor 180M may obtain a vibration signal. In some embodiments, the bone conduction sensor 180M may obtain a vibration signal of a vibration bone of a human vocal-cord part. The bone conduction sensor 180M may also be in contact with a body pulse to receive a blood pressure beating signal. In some embodiments, the bone conduction sensor 180M may also be disposed in the headset, to obtain a bone conduction headset. The audio module 170 may obtain a voice signal through parsing based on the vibration signal that is of the vibration bone of the vocal-cord part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt and a touch vibration feedback. For example, touch operations performed on different applications (for example, photographing and audio playing) may correspond to different vibration feedback effect. The motor 191 may also correspond to different vibration feedback effect for touch operations performed on different areas of the display 194. Different application scenarios (for example, time reminding, information receiving, an alarm clock, and a game) may also correspond to different vibration feedback effect. Touch vibration feedback effect may be further customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or removed from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be inserted into a same SIM card interface 195 at the same time. The plurality of cards may be of a same type or different types. The SIM card interface 195 may be compatible with different types of SIM cards. The SIM card interface 195 is also compatible with an external storage card. The electronic device 100 interacts with a network through the SIM card, to implement functions such as conversation and data communication. In some embodiments, the electronic device 100 uses an eSIM, that is, an embedded SIM card. The eSIM card may be embedded into the electronic device 100, and cannot be separated from the electronic device 100.

For ease of understanding, in the following embodiments of this application, an electronic device having the structure shown in FIG. 1 is used as an example to describe in detail the fatigue alerting method provided in embodiments of this application with reference to the accompanying drawings and application scenarios.

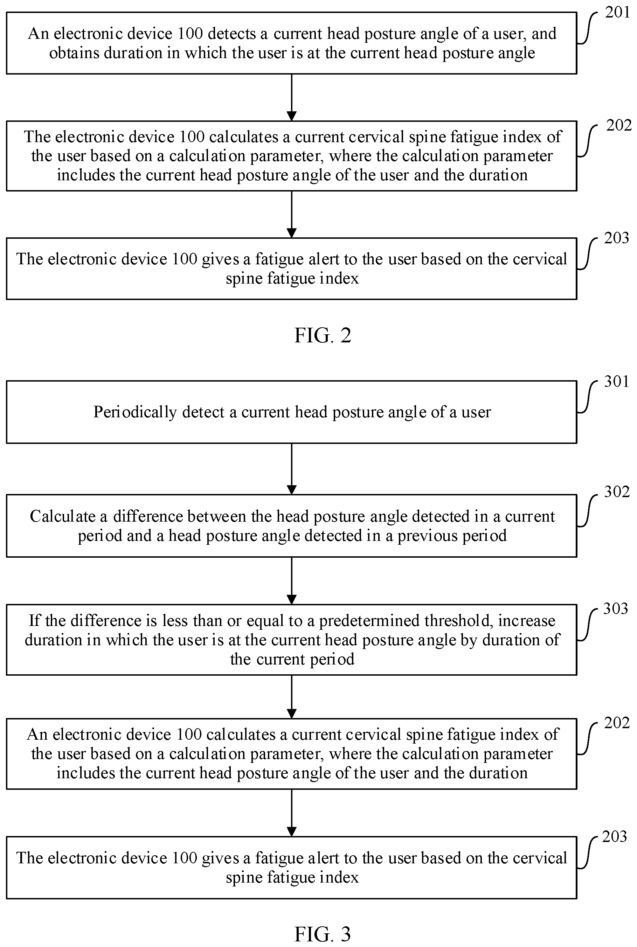

FIG. 2 is a flowchart of a fatigue alerting method according to an embodiment of this application. As shown in FIG. 2, the fatigue alerting method may include the following steps.

Step 201: An electronic device 100 detects a current head posture angle of a user, and obtains duration in which the user is at the current head posture angle.

In an implementation, the electronic device 100 may be a head-wearable device worn by the user, and the head-wearable device includes an inertial sensor 180H. In this way, the detecting a current head posture angle of a user may be: The electronic device 100 obtains inertial sensor data; and determines the current head posture angle of the user based on the inertial sensor data. In other words, in this implementation, when the electronic device 100 is the head-wearable device worn by the user, and the head-wearable device includes the inertia sensor 180H, the electronic device 100 may obtain the data detected by the inertia sensor 180H, and then determine the current head posture angle of the user based on the data detected by the inertia sensor 180H.

In another implementation, the detecting a current head posture angle of a user may be: The electronic device 100 obtains data of a user head image photographed by a camera; and determines the current head posture angle of the user based on the data of the user head image. The camera may be disposed in the electronic device 100, or the camera is communicatively connected to the electronic device 100. For example, when the electronic device 100 is a smartphone, and the user is in an indoor environment, a camera in the environment in which the user is located may photograph a user head image. Then, the smartphone obtains, through a communication connection to the camera, data of the user head image photographed by the camera, and detects the current head posture angle of the user based on the data of the user head image. Alternatively, when the electronic device 100 is a smart television, and a head of the user is within a photographing range of a camera of the smart television, the camera of the smart television may photograph a user head image, and then the smart television obtains data of the user head image photographed by the camera, to determine the current head posture angle of the user based on the data of the user head image.

In addition, it should be noted that, in this implementation, the data of the user head image photographed by the camera may include image data and/or video data.

Step 202: The electronic device 100 calculates a current cervical spine fatigue index of the user based on a calculation parameter, where the calculation parameter includes the current head posture angle of the user and the duration.

The cervical spine fatigue index is measured by a scientific experiment in combination with a subjective feeling of the user and an objective muscle (erector spinal muscle) electromyography index, and is used to measure a cervical spine fatigue degree index of the user.

The calculation parameter further includes basic information of the user, and the basic information of the user includes one or a combination of the following: an age, a gender, a height, and a weight of the user. Certainly, the basic information of the user may further include other information, for example, a residence place of the user, and the information included in the basic information of the user is not limited in this embodiment.

In other words, in this embodiment, the electronic device 100 may calculate the current cervical spine fatigue index of the user based on the current head posture angle of the user and the duration in which the user is at the current head posture angle; or the electronic device 100 may calculate the current cervical spine fatigue index of the user based on the current head posture angle of the user, the duration in which the user is at the current head posture angle, and the basic information of the user.

During specific implementation, the electronic device 100 may calculate the current cervical spine fatigue index of the user based on the foregoing calculation parameter by using a regression model. Simply speaking, the electronic device 100 inputs the foregoing calculation parameter into the regression model, performs calculation by using the regression model, and finally obtains the cervical spine fatigue index output by the regression model.

Step 203: The electronic device 100 gives a fatigue alert to the user based on the cervical spine fatigue index.

In the fatigue alerting method, the electronic device 100 detects the current head posture angle of the user, obtains the duration in which the user is at the current head posture angle, calculates the current cervical spine fatigue index of the user based on the calculation parameter, and gives the fatigue alert to the user based on the cervical spine fatigue index. In this way, the head posture angle of the user can be detected, the cervical spine fatigue index of the user is determined with reference to the duration in which the user is at the current head posture angle, and the fatigue alert can be given to the user based on the cervical spine fatigue index of the user, so that fatigue alerting is triggered more scientifically.

FIG. 3 is a flowchart of a fatigue alerting method according to another embodiment of this application. As shown in FIG. 3, in the embodiment shown in FIG. 2 of this application, step 201 may include the following steps.

Step 301: Periodically detect a current head posture angle of a user.

Step 302: Calculate a difference between the head posture angle detected in a current period and a head posture angle detected in a previous period.

Step 303: If the difference is less than or equal to a predetermined threshold, increase duration in which the user is at the current head posture angle by duration of the current period.

The preset threshold may be autonomously set during specific implementation based on system performance and/or an implementation requirement. This embodiment does not limit a value of the preset threshold. For example, the preset threshold may be 5.

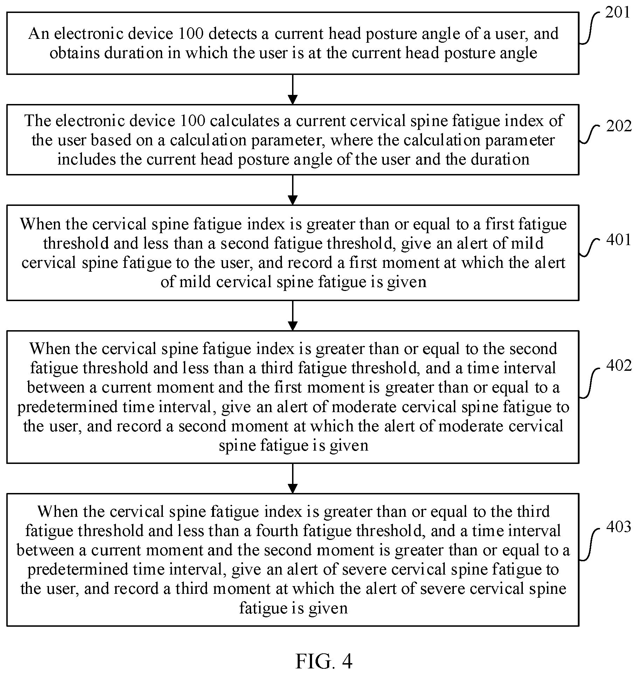

FIG. 4 is a flowchart of a fatigue alerting method according to another embodiment of this application. As shown in FIG. 4, in the embodiment shown in FIG. 2 of this application, step 203 may include the following steps.

Step 401: When the cervical spine fatigue index is greater than or equal to a first fatigue threshold and less than a second fatigue threshold, give an alert of mild cervical spine fatigue to the user, and record a first moment at which the alert of mild cervical spine fatigue is given.

Step 402: When the cervical spine fatigue index is greater than or equal to the second fatigue threshold and less than a third fatigue threshold, and a time interval between a current moment and the first moment is greater than or equal to a predetermined time interval, give an alert of moderate cervical spine fatigue to the user, and record a second moment at which the alert of moderate cervical spine fatigue is given.

Step 403: When the cervical spine fatigue index is greater than or equal to the third fatigue threshold and less than a fourth fatigue threshold, and a time interval between a current moment and the second moment is greater than or equal to a predetermined time interval, give an alert of severe cervical spine fatigue to the user, and record a third moment at which the alert of severe cervical spine fatigue is given.

Values of the first fatigue threshold, the second fatigue threshold, and the third fatigue threshold may be autonomously set during specific implementation based on system performance and/or an implementation requirement. The values of the first fatigue threshold, the second fatigue threshold, and the third fatigue threshold are not limited in embodiments. Similarly, a value of the predetermined time interval may be set during specific implementation based on system performance and/or the implementation requirement. The value of the predetermined time interval is not limited in embodiments.

In this embodiment, the cervical spine fatigue index is used as a determining basis for alert triggering, and fatigue alerting is triggered scientifically, user-friendly, and in line with user perception. In addition, after the alerting is triggered, whether the user relaxes is continuously monitored, and then whether higher-level alerting is triggered is determined based on the fatigue index. This implements multi-level alerting and gradual deepening, and explicitly alerts the user to a current cervical spine health state.

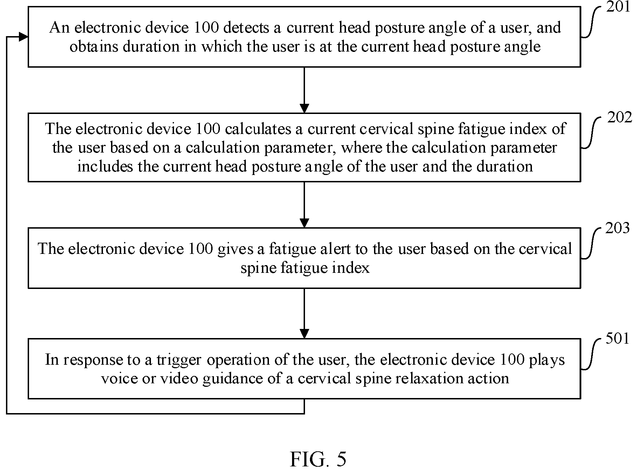

FIG. 5 is a flowchart of a fatigue alerting method according to another embodiment of this application. As shown in FIG. 5, in the embodiment shown in FIG. 2 of this application, after step 203, the method may further include the following step.

Step 501: In response to a trigger operation of the user, the electronic device 100 plays voice or video guidance of a cervical spine relaxation action.

The trigger operation may be preset, and the trigger operation may include one or a combination of the following: double-tapping the head-wearable device, holding the head up for predetermined duration, continuously nodding for a predetermined quantity of times, and voice inquiry. Certainly, the trigger operation may further include an operation in another form, which is not limited in this embodiment. Values of the predetermined duration and the predetermined quantity of times may be set during specific implementation. The predetermined duration and the predetermined quantity of times are not limited in this embodiment. For example, the predetermined duration may be 2 seconds, and the predetermined quantity of times may be 2 times.

In this embodiment, in response to the trigger operation of the user, the electronic device 100 may play voice or video guidance of the cervical spine relaxation action, so that the user obtains relaxation guidance in time, thereby relieving cervical spine fatigue.

Further, in this embodiment, after step 501, the electronic device 100 may reset the duration in which the user is at the current head posture angle, and return to continue to perform step 201 and subsequent steps.

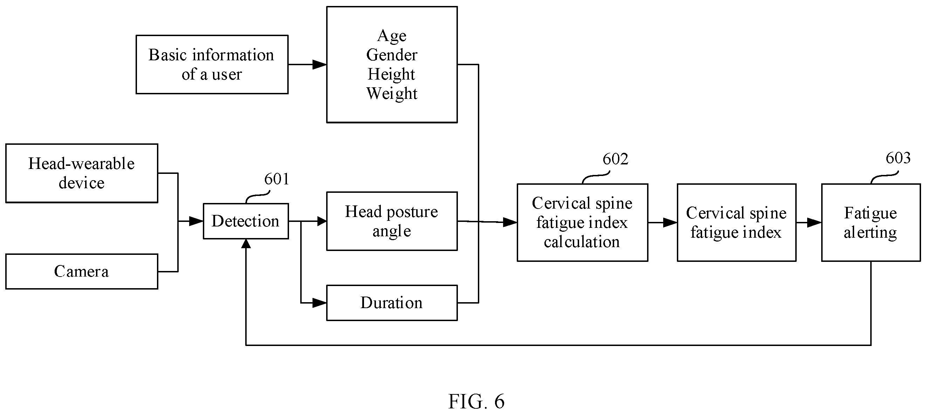

FIG. 6 is a flowchart of a fatigue alerting method according to another embodiment of this application. As shown in FIG. 6, the fatigue alerting method may include the following steps.

Step 601: An electronic device 100 detects a current head posture angle of a user, and obtains duration in which the user is at the current head posture angle.

Specifically, when the electronic device 100 is a head-wearable device worn by the user, and the head-wearable device includes an inertia sensor 180H, the electronic device 100 may obtain inertia sensor data; and determine the current head posture angle of the user based on the inertia sensor data; or the electronic device 100 may obtain data of a user head image photographed by a camera; and determine the current head posture angle of the user based on the data of the user head image.

Step 602: The electronic device 100 calculates a current cervical spine fatigue index of the user based on the current head posture angle of the user and the duration. In addition, when calculating the cervical spine fatigue index, the electronic device 100 may further perform calculation with reference to basic information of the user in addition to the current head posture angle of the user and the duration.

Step 603: The electronic device gives a fatigue alert to the user based on the cervical spine fatigue index.

Further, after the fatigue alert is given, the electronic device 100 returns to perform step 601, and continues to detect the current head posture angle of the user.

It can be understood that some or all of the steps or operations in the foregoing embodiments are merely examples, and other operations or variants of various operations may be further performed in embodiments of this application. In addition, the steps may be performed in another order different from that presented in the foregoing embodiments, and not all the operations in the foregoing embodiments may be performed.

It may be understood that, to implement the foregoing functions, the electronic device includes corresponding hardware and/or software modules for performing the functions. Algorithm steps in the examples described with reference to embodiments disclosed in this application can be implemented by hardware or a combination of hardware and computer software in this application. Whether a function is performed by hardware or hardware driven by computer software depends on particular applications and design constraints of the technical solutions. A person skilled in the art may use different methods to implement the described functions for each specific application with reference to embodiments. However, it should not be considered that the implementation goes beyond the scope of this application.

In this embodiment, the electronic device may be divided into functional modules based on the foregoing method embodiments. For example, each functional module may be obtained through division based on each corresponding function, or two or more functions may be integrated into one module. The integrated module may be implemented in a form of hardware. It should be noted that module division in embodiments is an example and is merely logical function division. In practice, there may be another division manner.

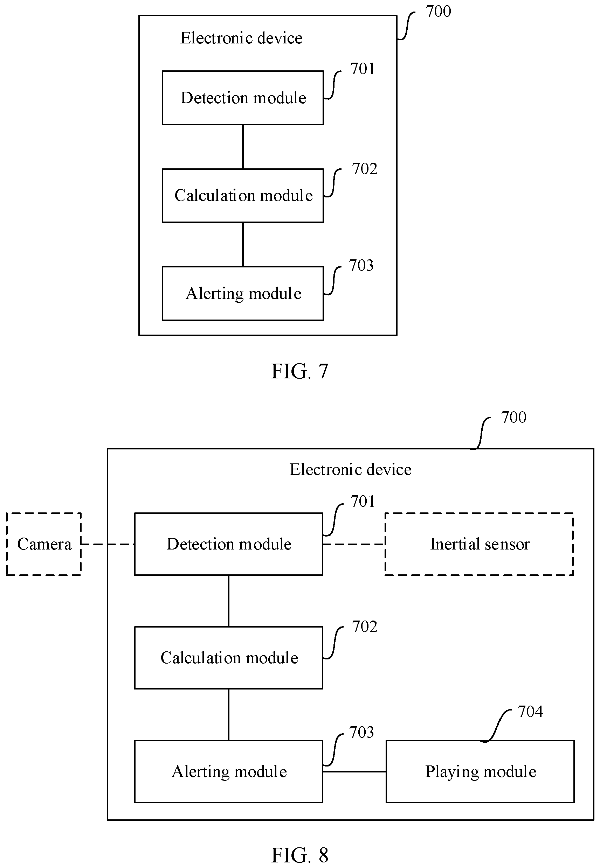

FIG. 7 is a diagram of a structure of an electronic device according to another embodiment of this application. When each functional module is obtained through division based on each corresponding function, FIG. 7 is a diagram of possible composition of an electronic device 700 in the foregoing embodiment. As shown in FIG. 7, the electronic device 700 may include: a detection module 701, a calculation module 702, and an alerting module 703.

The detection module 701 is configured to detect a current head posture angle of a user, and obtain duration in which the user is at the current head posture angle.

The calculation module 702 is configured to calculate a current cervical spine fatigue index of the user based on a calculation parameter, where the calculation parameter includes the current head posture angle of the user and the duration.

The alerting module 703 is configured to give a fatigue alert to the user based on the cervical spine fatigue index.

It should be noted that all related content of the steps in the method embodiment shown in FIG. 2 in this application may be cited in function description of corresponding functional modules. Details are not described herein again.

The electronic device 700 provided in this embodiment is configured to perform the fatigue alerting method provided in the embodiment shown in FIG. 2 of this application, and therefore can achieve same effect as the foregoing method.

FIG. 8 is a diagram of a structure of an electronic device according to another embodiment of this application. In comparison with the electronic device shown in FIG. 7, a difference lies in that in an implementation, the electronic device 700 shown in FIG. 8 may be a head-wearable device worn by a user, and the head-wearable device includes an inertia sensor.

In this way, the detection module 701 is specifically configured to obtain inertia sensor data, and determine a current head posture angle of a user based on the inertia sensor data.

In another implementation, the detection module 701 is specifically configured to obtain data of a user head image photographed by a camera; and determine the current head posture angle of the user based on the data of the user head image. The camera may be disposed in the electronic device 700, or the camera is communicatively connected to the electronic device 700.

In this embodiment, the calculation parameter further includes basic information of the user, and the basic information of the user includes one or a combination of the following: an age, a gender, a height, and a weight of the user.

In this embodiment, the detection module 701 is specifically configured to periodically detect the current head posture angle of the user, calculate a difference between the head posture angle detected in a current period and a head posture angle detected in a previous period, and if the difference is less than or equal to a preset threshold, increase the duration in which the user is at the current head posture angle by duration of the current period.

In this embodiment, the alerting module 703 is specifically configured to: when the cervical spine fatigue index is greater than or equal to a first fatigue threshold and less than a second fatigue threshold, give an alert of mild cervical spine fatigue to the user, and record a first moment at which the alert of mild cervical spine fatigue is given; when the cervical spine fatigue index is greater than or equal to the second fatigue threshold and less than a third fatigue threshold, and a time interval between a current moment and the first moment is greater than or equal to a predetermined time interval, give an alert of moderate cervical spine fatigue to the user, and record a second moment at which the alert of moderate cervical spine fatigue is given; and when the cervical spine fatigue index is greater than or equal to the third fatigue threshold and less than a fourth fatigue threshold, and a time interval between a current moment and the second moment is greater than or equal to a predetermined time interval, give an alert of severe cervical spine fatigue to the user, and record a third moment at which the alert of severe cervical spine fatigue is given.

Further, the electronic device 700 may further include a playing module 704.

The playing module 704 is configured to: after that the alerting module 703 gives the fatigue alert to the user based on the cervical spine fatigue index, in response to a trigger operation of the user, play voice or video guidance of a cervical spine relaxation action.

It should be understood that the electronic device 700 may correspond to the electronic device 100 shown in FIG. 1. A function of the detection module 701 may be implemented by the processor 110 in combination with the IMU 180H or the camera 193 in the electronic device 100 shown in FIG. 1, and functions of the calculation module 702, the alerting module 703, and the playing module 704 may be implemented by the processor 110 and the audio module 170 in the electronic device 100 shown in FIG. 1.

When an integrated unit is used, the electronic device 700 may include a processing module, a storage module, and a communication module.

The processing module may be configured to control and manage an action of the electronic device 700. For example, the processing module may be configured to support the electronic device 700 to perform the steps performed by the detection module 701, the calculation module 702, the alerting module 703, and the playing module 704. The storage module may be configured to support the electronic device 700 to store program code, data, and the like. The communication module may be configured to support the electronic device 700 to communicate with another device.

The processing module may be a processor or a controller, and may implement or execute various example logic blocks, modules, and circuits described with reference to content disclosed in this application. The processor may alternatively be a combination for implementing a computing function, for example, a combination including one or more microprocessors or a combination of a digital signal processor (digital signal processor, DSP) and a microprocessor. The storage module may be a memory. The communication module may be specifically a device, for example, a radio frequency circuit, a Bluetooth chip, and/or a Wi-Fi chip, that interacts with another electronic device.

In an embodiment, when the processing module is a processor, and the storage module is a memory, the electronic device 700 in this embodiment may be a device having the structure shown in FIG. 1.

An embodiment of this application further provides a computer-readable storage medium. The computer-readable storage medium stores a computer program. When the computer program is run on a computer, the computer is enabled to perform the methods provided in embodiments shown in FIG. 2 to FIG. 6 in this application.

An embodiment of this application further provides a computer program product. The computer program product includes a computer program. When the computer program is run on a computer, the computer is enabled to perform the methods provided in embodiments shown in FIG. 2 to FIG. 6 in this application.

In embodiments of this application, "at least one" means one or more, and "a plurality of" means two or more. In addition, "and/or" describes an association relationship between associated objects and represents that three relationships may exist. For example, A and/or B may represent the following cases: Only A exists, both A and B exist, and only B exists. A and B may be singular or plural. The character "/" generally indicates an "or" relationship between the associated objects. "At least one of the following" and similar expressions refer to any combination of these terms, including any combination of single or plural terms. For example, at least one item of a, b, or c may indicate a, b, c, a and b, a and c, b and c, or a, b, and c, where a, b, and c may be singular or plural.

A person of ordinary skill in the art may be aware that the units and algorithm steps described in embodiments disclosed in this specification can be implemented by a combination of electronic hardware, computer software, and electronic hardware. Whether these functions are performed by hardware or software depends on particular applications and design constraints of the technical solutions. A person skilled in the art may use different methods to implement the described functions for each specific application. However, it should not be considered that the implementation goes beyond the scope of this application.

It may be clearly understood by a person skilled in the art that, for the purpose of convenient and brief description, for a detailed working process of the foregoing system, apparatus, and unit, refer to a corresponding process in the foregoing method embodiments. Details are not described herein again.

In several embodiments provided in this application, when any function is implemented in a form of software functional unit and sold or used as an independent product, the function may be stored on a computer-readable storage medium. Based on such an understanding, the technical solutions of this application essentially, or the part contributing to the conventional technology, or some of the technical solutions may be implemented in a form of software product. The software product is stored in a storage medium, and includes several instructions for enabling a computing device (which may be a personal computer, a server, or a network device) to perform all or some of the steps of the methods described in embodiments of this application. The foregoing storage medium includes any medium that can store program code, for example, a USB flash drive, a removable hard disk, a read-only memory (read-only memory, ROM), a random access memory (random access memory, RAM), a magnetic disk, or an optical disc.

The foregoing descriptions are merely specific implementations of embodiments of this application. Any variation or replacement readily figured out by a person skilled in the art within the technical scope disclosed in this application shall fall within the protection scope in embodiments of this application. Therefore, the protection scope of this application shall be subject to the protection scope of the claims.

## Claims

1. A fatigue alerting method, comprising:
detecting, by an electronic device, a current head posture angle of a user, and obtaining duration in which the user is at the current head posture angle;
calculating a current cervical spine fatigue index of the user based on a calculation parameter, wherein the calculation parameter comprises the current head posture angle of the user and the duration; and
giving a fatigue alert to the user based on the cervical spine fatigue index.

2. The method according to claim 1, wherein the electronic device comprises a head-wearable device worn by the user, and the head-wearable device comprises an inertial sensor; and
detecting the current head posture angle of the user comprises:
obtaining inertial sensor data; and
determining the current head posture angle of the user based on the inertial sensor data.

3. The method according to claim 1, wherein detecting the current head posture angle of the user comprises:
obtaining data of a user head image photographed by a camera, wherein the camera is disposed in the electronic device, or the camera is communicatively connected to the electronic device; and
determining the current head posture angle of the user based on the data of the user head image.

4. The method according to claim 1, wherein the calculation parameter further comprises basic information of the user, and the basic information of the user comprises one or a combination of the following: an age, a gender, a height, and a weight of the user.

5. The method according to claim 1, wherein detecting the current head posture angle of the user comprises:
periodically detecting the current head posture angle of the user, wherein
obtaining the duration in which the user is at the current head posture angle comprises:
calculating a difference between the head posture angle detected in a current period and a head posture angle detected in a previous period; and
if the difference is less than or equal to a predetermined threshold, increasing the duration in which the user is at the current head posture angle by duration of the current period.

6. The method according to any one of claims 1 to 5, wherein giving the fatigue alert to the user based on the cervical spine fatigue index comprises:
when the cervical spine fatigue index is greater than or equal to a first fatigue threshold and less than a second fatigue threshold, giving an alert of mild cervical spine fatigue to the user, and recording a first moment at which the alert of mild cervical spine fatigue is given;
when the cervical spine fatigue index is greater than or equal to the second fatigue threshold and less than a third fatigue threshold, and a time interval between a current moment and the first moment is greater than or equal to a predetermined time interval, giving an alert of moderate cervical spine fatigue to the user, and recording a second moment at which the alert of moderate cervical spine fatigue is given; and
when the cervical spine fatigue index is greater than or equal to the third fatigue threshold and less than a fourth fatigue threshold, and a time interval between a current moment and the second moment is greater than or equal to a predetermined time interval, giving an alert of severe cervical spine fatigue to the user, and recording a third moment at which the alert of severe cervical spine fatigue is given.

7. The method according to any one of claims 1 to 5, wherein after giving the fatigue alert to the user based on the cervical spine fatigue index, the method further comprises:
in response to a trigger operation of the user, playing voice or video guidance of a cervical spine relaxation action.

8. An electronic device, comprising:
a detection module, configured to: detect a current head posture angle of a user, and obtain duration in which the user is at the current head posture angle;
a calculation module, configured to calculate a current cervical spine fatigue index of the user based on a calculation parameter, wherein the calculation parameter comprises the current head posture angle of the user and the duration; and
an alerting module, configured to give a fatigue alert to the user based on the cervical spine fatigue index.

9. An electronic device, comprising:
one or more processors, a memory, a plurality of applications, and one or more computer programs, wherein the one or more computer programs are stored in the memory, the one or more computer programs comprise instructions, and when the instructions are executed by the electronic device, the electronic device is enabled to perform the method according to any one of claims 1 to 7.

10. A computer-readable storage medium, wherein the computer-readable storage medium stores a computer program, and when the computer program is run on a computer, the computer is enabled to perform the method according to any one of claims 1 to 7.
